# NEUE EUROPÄISCHE PATENTSCHRIFT

(11) **EP 1 795 186 B2**
(45) Veröffentlichungstag und Bekanntmachung des Hinweises auf die Entscheidung über den Einspruch: **06.12.2017**
(45) Hinweis auf die Patenterteilung: 23.10.2013
(21) Anmeldenummer: 06022985.3
(22) Anmeldetag: 04.11.2006
(51) Int. Cl.: A61K 9/16, A61K 9/20, A61K 9/50

(54) **Flupirtin umfassende Arzneimittelzubereitung mit kontrollierter Wirkstofffreisetzung**
Flupirtin comprising medicament formulation with a controlled release of the active agent
Formulation medicamenteuse contenant Flupirtin à libération controlée du principe actif

(30) Priorität: 08.11.2005 DE 102005054610
(43) Veröffentlichungstag der Anmeldung: 13.06.2007
(73) Patentinhaber: TEVA GmbH, 89079 Ulm (DE)
(72) Erfinder: Terhaag, Bernd, 01796 Pirna (DE); Wolf, Joachim, 01445 Radebeul (DE); Qadan, Asal, 01099 Dresden (DE); Faustmann, Barbara, 01108 Dresden (DE)
(74) Vertreter: Lang, Johannes

(56) Entgegenhaltungen:
- EP-A1- 0 615 754
- WO-A-2004/012700
- WO-A-2005/000306
- US-A1- 2006 240 043
- FLORENCE LECOMTE ET AL: "pH-Sensitive Polymer Blends Used as Coating Materials to Control Drug Release from Spherical Beads: Elucidation of the Underlying Mass Transport Mechanisms" PHARMACEUTICAL RESEARCH, KLUWER ACADEMIC PUBLISHERS-PLENUM PUBLISHERS, NE, Bd. 22, Nr. 7, 1. Juli 2005 (2005-07-01), Seiten 1129-1141, XP019370885 ISSN: 1573-904X
- BODMEIER R: "Tableting of coated pellets" EUROPEAN JOURNAL OF PHARMACEUTICS AND BIOPHARMACEUTICS, ELSEVIER SCIENCE PUBLISHERS B.V., AMSTERDAM, NL, Bd. 43, Nr. 1, Januar 1997 (1997-01), Seiten 1-8, XP004256852 ISSN: 0939-6411
- DEBUNNE ANN ET AL: "Compaction of enteric-coated pellets: influence of formulation and process parameters on tablet properties and in vivo evaluation" EUROPEAN JOURNAL OF PHARMACEUTICAL SCIENCES, Bd. 22, Nr. 4, Juli 2004 (2004-07), Seiten 305-314, XP002449822 ISSN: 0928-0987
- HU ET AL: "Preparation and in vitro/in vivo evaluation of sustained-release metformin hydrochloride pellets" EUROPEAN JOURNAL OF PHARMACEUTICS AND BIOPHARMACEUTICS, MEDPHARM SCIENTIFIC PUBL., STUTTGART, DE, Bd. 64, Nr. 2, Oktober 2006 (2006-10), Seiten 185-192, XP005625734 ISSN: 0939-6411

## Beschreibung

Die Erfindung betrifft feste Arzneimittelzubereitungen umfassend Flupirtin oder seine physiologisch verträglichen Salze als Wirkstoff, wobei mindestens ein Teil des Flupirtins oder seiner physiologisch verträglichen Salze als Wirkstoffformulierung mit verzögerter Wirkstofffreisetzung vorliegt, sowie Verfahren zu deren Herstellung.

Flupirtin ist ein überwiegend zentral wirksames Analgetikum, welches zusätzlich erhöhte Muskelverspannungen vermindert. Das wesentliche therapeutische Einsatzgebiet von Flupirtin ist die Behandlung chronischer Schmerzen wie z. B. muskuloskeletale Schmerzen, postoperative Schmerzen, Schmerzen nach Verletzungen oder Tumor bedingte Schmerzen. Durch einen speziellen Wirkungsmechanismus am neuronalen K+-Kanal (Selective Neuronal Potassium Channel Opener [SNEPCO]) verringert Flupirtin die erhöhte Erregbarkeit und wirkt indirekt als NMDA- Antagonist. Dadurch wird die Zunahme der intrazellulären Ca++-Konzentration verringert.

Flupirtin wird - üblicherweise in Form seines physiologisch verträglichen Salzes Flupirtinmaleat - oral, rektal oder parenteral verabreicht. Die üblichen Formulierungen für die Verabreichung von Flupirtin führen zu einer raschen Freisetzung des Wirkstoffs, so dass seine analgetische Wirkung schnell einsetzt. Zugleich beobachtet man ein rasches Abklingen der Wirkung. Somit erfordert die Behandlung starker chronischer Schmerzen mit Flupirtin die Verabreichung des Arzneimittels in relativ kurzen Abständen, um so eine ausreichende Wirkstoffkonzentration im Blutplasma des Patienten zu gewährleisten. Bei oraler Applikation werden in der Regel daher dreimal täglich 100 bis 200 mg Flupirtinmaleat verabreicht, bei rektaler Applikation drei bis viermal täglich 150 mg. Typische Nebenwirkungen von Flupirtin sind Müdigkeit, Schwindel, Magen/Darm-Beschwerden und Obstipation.

Die Notwendigkeit der häufigen Wirkstoffapplikation führt jedoch leicht zu Fehlern bei der Einnahme sowie zu unerwünschten Plasmakonzentrationsschwankungen, was der Patientencompliance und dem therapeutischen Nutzen abträglich ist, insbesondere bei der Behandlung chronischer Schmerzzustände. Eine pharmazeutische Darreichungsform mit verzögerter Freisetzung (Retardformulierung) des Wirkstoffs Flupirtin, die nur noch ein- bis zweimal am Tag verabreicht werden muss, ist daher wünschenswert.

Aus EP 0615754 B1 sind feste Arzneimittelzubereitungen bekannt, die den Wirkstoff Flupirtin oder seine physiologisch verträglichen Salze und eine Retardkomponente enthalten und aus denen der Wirkstoff Flupirtin kontrolliert freigesetzt wird. Die Wirkstofffreisetzung erfolgt Matrix- bzw. pH-kontrolliert.

Als Retardkomponente wird in EP 061574 B1 Polyacrylsäure (Carbopol®934) verwendet. Dabei handelt es sich um einen hydrophilen Matrixbildner. Die Kontrolle der Wirkstofffreisetzung erfolgt durch Quellung des Matrixbildners. Die Wirkstofffreisetzung ist somit Matrix-kontrolliert, d.h. die Freisetzung erfolgt über die gesamte Tablette (single unit dosage form) durch den mit der Zeit stattfindenden Abbau der Matrix. Bei single unit dosage Formen führt eine Teilung der Tablette über die Änderung des Verhältnisses Oberfläche zu Volumen und einer veränderten Diffusionsgeschwindigkeit zu einer veränderten Freisetzungskinetik.

Weiterhin wird in EP 0615754 B1 eine Mischung aus Schellack und Eudragit®L als Retardkomponente eingesetzt. Schellack/Eudragit® L100 dient in einer galenischen Zubereitung als Magensaft-resistente Komponente. Die kontrollierte Wirkstofffreisetzung erfolgt dabei durch die Erhöhung des pH-Wertes im Darm. Das verwendete Flupirtinmaleat ist jedoch in Salzsäure (Magensaft) löslich und die Herstellung einer Magensaft-resistenten galenisch modifizierten Zubereitung somit für die Resorption des Wirkstoffes kontraproduktiv.

Trotz Verwendung der Magensaft-resistenten Komponente kommt es innerhalb der ersten Stunde außerdem bereits zur Freisetzung von 43 Prozent des enthaltenen Wirkstoffes in 0,1 N HCl. Dies zeigt, dass Schellack/Eudragit® L100 in dieser Arzneimittelzubereitung wenig retardierend wirkt und wohl nur als Bindemittel dient.

Die WO 97/14415 A1 offenbart eine pharmazeutische Formulierung umfassend einen NMDA Rezeptor Antagonisten, wobei der Wirkstoff sowohl in Form mit sofortiger Wirkstofffreisetzung als auch in Form mit verzögerter Freisetzung vorliegt. Die Formulierungen werden dabei hergestellt, indem wirkstoffhaltige Granulate für die sofortige Freisetzung mit wirkstoffhaltigen, mit einer Retardkomponente überzogenen, Pellets für die verzögerte Freisetzung vermischt und in Kapseln abgefüllt werden. Flupirtin ist jedoch kein direkter NMDA Rezeptor Antagonist und weist sehr spezifische physikalische Eigenschaften auf, die nicht vergleichbar sind mit der Allgemeinheit der NMDA Rezeptor Antagonisten.

WO2004/012700 betrifft eine Arzneimittelformulierung, umfassend einen aktiven Bestandteil hoher Löslichkeit in hoher Dosis und einen aktiven Bestandteil mittlerer Löslichkeit in geringer Dosis, so dass in Kombination eine retardierte Arzneimittelformulierung bereit gestellt wird.

Der Artikel von F. Lecomte et al., Pharmaceutical Research, Band 22, Nr. 7, Juli 2005, Seiten 1129-1141, betrifft die Retardierung von Verapamil-Hydrochlorid durch Beschichtung von Verapamil-Hydrochlorid enthaltenden Kügelchen.

Die Publikation von R. Bodmeier, European Journal of Pharmaceutics and Biopharmaceutics, Band 43, 1997, Seiten 1-8, betrifft die Kompaktierung von beschichteten Pellets zu stabilen Tabletten und beschreibt die dazu notwendigen Verfahrensparameter.

Die Publikation von A. Debunne et al., European Journal of Pharmaceutical Sciences, 22, 2004, Seiten 305-314, betrifft eine Untersuchung des Einflusses der Formulierung und der Kompressionsparameter auf die Eigenschaften von Piroxicam enthaltenden Tabletten.

Die EP615754 beschreibt Flupirtin enthaltende Arzneimittelzubereitungen mit kontrollierter Wirkstofffreigabe unter Verwendung einer Retardkomponente in Single Unit Dosage Form.

Auch weiterhin besteht daher ein Bedarf an verbesserten festen Arzneimittelzubereitungen von Flupirtin, die eine gleichmäßige und anhaltende Wirkstofffreisetzung über längere Zeit gewährleisten und daher nur noch ein bis zweimal am Tag eingenommen werden müssen.

Aufgabe der Erfindung ist es daher, eine feste Arzneimittelzubereitung zur oralen oder rektalen Anwendung mit kontrollierter Wirkstofffreisetzung, die als Wirkstoff Flupirtin oder seine physiologisch verträglichen Salze enthält, sowie Verfahren zu deren Herstellung bereitzustellen, wobei die Arzneimittelzubereitung einen langanhaltenden Effekt aufweist, so dass sie nur noch ein- bis zweimal täglich eingenommen werden muss. Zudem soll die Arzneimittelsicherheit (bessere Bioverfügbarkeit, selteneres Auftreten von Nebenwirkungen) verbessert und das Risiko des "dose dumping" vermieden werden.

Erfindungsgemäß wird die Aufgabe gelöst durch eine feste Arzneimittelzubereitung wie eingangs angeführt, wobei
a. die Wirkstoffformulierung mit verzögerter Wirkstofffreisetzung Wirkstoffkompaktate umfasst, die mit einer Retardkomponente, vorzugsweise gleichmässig, überzogen sind und gegebenenfalls pharmazeutisch übliche Hilfsstoffe umfassen, so dass eine gleichmässige Freisetzung des Flupirtins aus den Kompaktaten gewähleistet ist, und
b. die Wirkstoffkompaktate eine Teilchengröße von 160 bis 800 µm, vorzugsweise 250 bis 500 µm, aufweisen und bevorzugt sphärisch oder annähernd sphärisch sind. Die Teilchengrösse wird dabei gemäss der 2.9.12. Siebanalyse nach Ph. Eur. 5 gemessen.

Mit Retardkomponente überzogene Kompaktate umfassend Flupirtin wurden in keinem Stand der Technik beschrieben oder nahegelegt.

Die erfindungsgemäße Arzneimittelzubereitung mit kontrollierter Wirkstofffreisetzung enthält als Wirkstoff Flupirtin oder physiologisch verträgliche Flupirtinsalze. Physiologisch verträgliche Salze des Wirkstoffs Flupirtin sind im Sinne dieser Erfindung solche Salze des Wirkstoffs, die bei pharmazeutischer Verwendung bei Anwendung am Säugetier und/oder Menschen verträglich sind. Solche physiologisch verträglichen Salze können beispielsweise mit anorganischen oder organischen Säuren gebildet werden. Geeignete Salze sind beispielsweise Flupirtinhydrochlorid, Flupirtinmaleat und Flupirtin-D-Gluconat.

Die Mengenangaben in der vorliegenden Anmeldung beziehen sich bei der Angabe 'Flupirtin' stets auf die Basenform und sind bei Vorliegen eines Salzes entsprechend dem erhöhten Molekulargewicht umzurechnen.

In der erfindungsgemäßen Arzneimittelzusammensetzung liegt mindestens ein Teil des Flupirtins bzw. seiner physiologisch verträglichen Salze als Winkstoffformulierung mit verzögerter Wirkstofffreisetzung vor. Bestandteil der Erfindung ist somit auch eine Arzneimittelzubereitung, in der das gesamte Flupirtin bzw. seine physiologisch verträglichen Salze in der erfindungsgemäßen Arzneimittelzusammensetzung als Wirkstoffformulierung mit verzögerter Wirkstofffreisetzung vorliegen.

Zudem kann die Arzneimittelzubereitung neben Flupirtin weitere Wirkstoffe umfassen, vorzugsweise enthält sie jedoch nur Flupirtin. Selbstverständlich ist Flupirtin in einer pharmazeutisch wirksamen Menge vorgesehen.

Der Begriff "verzögerte Wirkstofffreisetzung" bedeutet im Rahmen der vorliegenden Erfindung eine in vitro Wirkstofffreisetzung von max. 75% über einen Zeitraum von mind. 4h. Diese in vitro Freisetzungsgeschwindigkeit des Wirkstoffes aus der Arzneimittelzubereitung wird dabei unter Anwendung der Ph. Eur. Paddle Method bei 100 U/min in einem Puffer (gemäss Ph. Eur.) bei einem pH-Wert von 6,8 bei 37°C und unter UV-spektrophotometrischen Detektion, gemessen.

Der als Wirkstoffformulierung mit verzögerter Wirkstofffreisetzung vorliegende Teil des Flupirtins oder seine physiologisch verträglichen Salze ist - gegebenenfalls unter Zusatz pharmazeutisch üblicher Hilfsstoffe - zu Wirkstoffkompaktaten kompaktiert.

Bei der Kompaktierung wird der Wirkstoff (Flupirtin bzw. seine physiologisch verträglichen Salze) gegebenenfalls unter Zugabe pharmazeutisch üblicher Hilfsstoffe in einer geeigneten Vorrichtung wie beispielsweise in einer Walzenpresse, in einem Walzenkompaktor, im Rollkompaktor oder in der Rollpresse im Spalt zwischen zwei sich gegensinnig drehenden glatten oder profilierten Walzen verdichtet. Aus dem dabei erzeugten Materialband, der sog. Schülpe, wird in einem Zerkleinerungs- und anschließend Siebkreislauf ein Wirkstoffkompaktat mit definierter Teilchengröße von 160 bis 800 µm, bevorzugt von 250 bis 500 µm, hergestellt.

Bei der Kompaktierung werden vorzugsweise hydraulische Kompaktierungskräfte von 3000 kN/m² bis 9000 kN/m² eingesetzt. Vorzugsweise wird eine Walzendrehzahl von 1 U/min bis 10 U/min verwendet. Die Schneckendrehzahl kann zwischen 10 U/min bis 70 U/min variiert werden.

Flupirtin weist eine nadelförmige Kristallstruktur auf. Aufgrund dieser Kristallstruktur kann Flupirtin ebenfalls nicht optimal und regelmässig mit einer Retardkomponente beschichtet werden. Vor allem die Kanten und Spitzen der Nadeln lassen sich nur schwer und unregelmässig befilmen. Durch die Kompaktierung ist es nun überraschenderweise gelungen, die physikalischen Eigenschaften des Flupirtins so zu optimieren, dass eine reproduzierbare und gleichmäßige Befilmung ermöglicht wird.

Vorteilhaft hat die beim Kompaktiervorgang durch die Walzen ausgeübte Presskraft einen vernachlässigbaren oder überhaupt keinen Einfluss auf das Freisetzungsprofil der verzögert freisetzenden Arzneimittelzubereitung. Die Kompaktierung wird zur Verbesserung der physikalischen Feststoffparameter wie z.B. Teilchengröße, Schüttvolumen, nicht aber zur Erzielung einer kontrollierten Wirkstofffreisetzung verwendet.

Der nicht-kompaktierte Wirkstoff Flupirtin bzw. seine physiologisch verträglichen Salze sind sehr voluminös und besitzen hohe Schütt- bzw. Stampfvolumina. So hat z.B. Flupirtinmaleat ein Schüttvolumen zwischen 7,5 und 10,5 ml/g und ein Stampfvolumen zwischen 4 und 5,5 ml/g. Die Teilchengröße des nicht-kompaktierten Wirkstoffes liegt zwischen 5 bis 100 µm mit einer sehr breiten Teilchengrößenverteilung. Die Teilchengrösse wird dabei gemäss der 2.9.12. Siebanalyse oder alternativ durch Mikroskopie gemäss 2.9.13. nach Ph. Eur. 5 gemessen. Eine reproduzierbare Umhüllung der nicht-kompaktierten Wirkstoffteilchen mit einer Retardkomponente ist bei einer solch breiten Teilchengrößenverteilung jedoch nicht möglich. Die spezifische Oberfläche des nicht-kompaktierten Wirkstoffs liegt zwischen 110 - 180 m²/l.

Zudem haben der nicht-kompaktierte Wirkstoff Flupirtin bzw. seine physiologisch verträglichen Salze schlechte Fließeigenschaften und weisen eine starke Neigung zur Brückenbildung auf. Daher ist der Wirkstoff nur ungenau zu dosieren. Aufgrund seiner Struktur und Sprödigkeit ist der Wirkstoff zudem schlecht verformbar. Die daraus resultierenden technischen Schwierigkeiten verhindern eine reproduzierbare Weiterverarbeitung des Wirkstoffes zu einer pharmazeutischen Darreichungsform wie z.B. insbesondere bei der Tablettierung.

Der kompaktierte Wirkstoff hingegen ist homogen und weist verbesserte Fließeigenschaften und ein verringertes Schüttvolumen auf. Genauso wird durch die Kompaktierung des Wirkstoffes Flupirtin bzw. seiner physiologisch verträglichen Salze sowohl die Teilchengrößenverteilung als auch die Oberfläche des Flupirtins, die zur Umhüllung mit der Retardkomponente zur Verfügung steht, genau definiert (spezifische Oberfläche 10 - 25 m²/l). Dies ermöglicht die gleichmäßige Befilmung des Wirkstoffes mit der Retardkomponente und ggf. Hilfsstoffen und somit ein definiertes Freisetzungsverhalten. Dies erlaubt eine genauere und reproduzierbare Dosierung des Wirkstoffes.

Durch das Kompaktieren des Wirkstoffes ggf. unter Zusatz pharmazeutisch üblicher Hilfsstoffe wird also eine bestimmte Teilchengrößenfraktion mit enger Teilchengrößenverteilung erhalten, die eine einheitliche Oberfläche aufweist. Ferner wird durch die Verringerung des Schüttvolumens und die Verbesserung der Fließfähigkeit ein Überziehen der Wirkstoffkompaktate mit der Retardkomponente unter reproduzierbaren Bedingungen möglich.

Die Wirkstoffkompaktate aus Flupirtin erlauben daher aufgrund der homogenen Größe und der Form des Flupirtins, der reproduzierbaren und einheitlichen Beschichtung des Flupirtins mit Retardkomponenten und der genauen Dosierung überraschenderweise eine gleichmäßige Freisetzung des Wirkstoffes aus den einzelnen Kompaktaten. Dies bedeutet, dass eine definierte, über einen längeren Zeitraum von z.B. 12 bis 24 h nach Einnahme der Arzneimittelzubereitung ein konstanter Wirkspiegel durch verzögerte Abgabe des Wirkstoffes aus der Arzneiform an den Körper erzielt wird. Es kommt weder zu Dosierungsschwankungen noch zum "dose dumping", und es wird hierdurch im Vergleich zu den herkömmlichen Flupirtin-Arzneiformen eine Verringerung der Nebenwirkungen erreicht.

Da die Freisetzung aus den einzelnen Kompaktaten erfolgt, gehört die erfindungsgemäße Arzneimittelzubereitung zu der Gruppe der "Multiple Unit Dosage Forms" und nicht zu den "Single Unit Dosage Forms". Da einzelne Kompaktate mit der Retardierungskomponente überzogen sind im Vergleich zur Beschichtung der gesamten Tablette gemäss dem Stand der Technik, wird es erfindungsgemäss nun ermöglicht, Flupirtin in zerteilbare Arzneimittelzubereitungen vorzusehen. Z.B. kann eine Tablette umfassend Flupirtin hergestellt werden, die nach Belieben zerteilbar ist, ohne dass die Retard-Schicht zerstört wird und ohne dass das Freisetzungsprofil wesentlich beeinflusst wird. Damit wird ebenfalls das "dose-dumping" verhindert.

Die Kompaktierung von Flupirtin bzw. seiner physiologisch verträglichen Salze kann mit oder ohne den sonst üblichen Zusatz von Hilfsstoffen erfolgen. Die als Hilfsstoffe bei der Kompaktierung von Flupirtin bzw. seiner physiologisch verträglichen Salze eingesetzten Substanzen weisen vorzugsweise bestimmte physiko-chemische Eigenschaften wie z.B. hohe Plastizität, akzeptable Fließfähigkeit und gute Verpressbarkeit auf. Ferner dürfen sie nicht mit anderen Substanzen reagieren und dürfen nicht toxisch sein. Als Hilfsstoff bei der Kompaktierung kann z.B. mikrokristalline Cellulose eingesetzt werden.

Durch die Einstellung der oben genannten Prozessparameter bei der Kompaktierung können auch ohne Zusatz von Hilfsstoffen sphärische oder annähernd sphärische Wirkstoffkompaktate mit enger Teilchengrößenverteilung und definierter Oberfläche erhalten werden, die ein reproduzierbares Überziehen mit Retardkomponente gewährleisten. Somit ist auch bei Kompaktierung ohne Zusatz von Hilfsstoffen eine gleichmäßige und kontrollierte Freisetzung von Flupirtin bzw. seinen physiologisch verträglichen Salzen möglich, wobei auch ein sog. "dose dumping" vermieden werden kann.

Die erfindungsgemäße Arzneimittelzubereitung muss aufgrund der verzögerten Wirkstofffreisetzung nur noch ein- bis zweimal täglich vom Patienten eingenommen werden. Die erfindungsgemässe Arzneimittelzubereitung erlaubt demnach eine Schmerztherapie, bei der Flupirtin bzw. seine physiologisch verträglichen Salze nur noch einmal täglich, z.B. im Abstand von 24 h, oder zweimal täglich, vorzugsweise im Abstand von 12 Stunden, verabreicht werden muss, um eine ausreichende Plasmakonzentration des Wirkstoffs zu gewährleisten. Eine entsprechende Wirkdauer und die Aufrechterhaltung ausreichender WirkSpiegel im Plasma werden durch experimentelle Untersuchungen belegt.

Dadurch lässt sich die Akzeptanz des Arzneimittels durch den Patienten ("compliance") wesentlich verbessern, denn gerade Patienten mit starken Schmerzen, zum Beispiel Patienten mit chronischen Rückenschmerzen oder Patienten mit Tumorschmerzen, empfinden die seltenere Arzneimittelgabe sowie die längere Wirkungsdauer insbesondere bei nächtlichen Schmerzen als vorteilhaft.

Ferner wird die erfindungsgemäße Zusammensetzung besser toleriert als die herkömmlichen schnell freisetzenden Formulierungen von Flupirtin mit gleicher Wirkstoffkonzentration. Die Flupirtin-typischen Nebenwirkungen - wie Müdigkeit und Schwindelgefühle - treten bei Anwendung der erfindungsgemäßen Arzneimittelzubereitung deutlich seltener und in geringerer Intensität auf.

Bei der erfindungsgemäßen "Multiple Unit Dosage Form" erfolgt die Freisetzung des Wirkstoffes zudem weitgehend unabhängig von Art und Menge der vom Patienten aufgenommenen Nahrung und es wird selbst nach Nahrungsaufnahme eine sehr gleichmässige Wirkstofffreisetzung erzielt. Die erfindungsgemässe Arzneimittelzubereitung ist zu jeder für Flupirtin geeigneten Anwendung vorgesehen, vorzugsweise für die orale oder rektale Anwendung.

Ausserordentlich überraschend ist auch die Beobachtung, dass bei Verabreichung der erfindungsgemässen Arzneimittelzubereitung mit verzögerter Wirkstofffreisetzung bei menschlichen Versuchspersonen eine im Vergleich zu Formulierungen mit sofortiger Wirkstofffreisetzung nur geringfügig reduzierte Bioverfügbarkeit erreicht wird.

Gemäss einer vorteilhaften Arzneimittelzubereitung weisen die Wirkstoffkompaktate ein Schüttvolumen von unter 5 ml/g, vorzugsweise unter 3 ml/g, noch bevorzugter unter 2,5 ml/g, am meisten bevorzugt zwischen 0,8 und 2,5 ml/g, auf. Das Schüttvolumen der Kompaktate ist wesentlich geringer als das Schüttvolumen nicht kompaktierten Flupirtins, das etwa zwischen 7,5 und 10,5 ml/g beträgt. Das Wirkstoffkompaktat ist damit weniger voluminös, weist bessere Fliesseigenschaften auf und ist somit auf reproduzierbarere Weise verarbeitbar und genauer zu dosieren.

Vorzugsweise gewährleistet die Retardkomponente eine diffusionskontrollierte Freisetzung des Wirkstoffes. Die Freisetzung erfolgt daher unabhängig vom pH-Wert und nicht Matrix-kontrolliert. Sobald Flüssigkeit durch den Überzug der Retardkomponente diffundiert, quillt der Überzug und wird permeabler. Die Flüssigkeit im Innenraum löst das Flupirtinbzw. seine Salze auf, der Wirkstoff diffundiert durch den Überzug nach außen und wird freigesetzt. Die Wirkstofffreisetzung erfolgt dabei durch Diffusion aus den einzelnen überzogenen Wirkstoffkompaktaten.

Da die Freisetzung über die Diffusion des Wirkstoffes aus den mit der Retardkomponente überzogenen einzelnen Kompaktaten kontrolliert wird, wird eine besonders gleichmäßige anhaltende Freisetzung über längere Zeit gewährleistet, insbesondere im Vergleich zur pHkontrollierten und auch Matrix-kontrollierten Freisetzung. Dies führt dazu, dass bei einer therapeutisch wirksamen Dosierung nur noch ein- bis maximal zweimal am Tag eine Arzneimitteleinnahme benötigt wird und die Dosierung sicher erfolgt.

Besonders bevorzugt umfasst die Retardkomponente einen retardierenden Polymerfilm und ggf. übliche pharmazeutische Hilfsstoffe, vorzugsweise Trennmittel und Pigmente, wobei der retardierende Polymerfilm vorzugsweise zumindest ein Polymer oder Copolymer aus Acrylsäure, Acrylsäurederivaten, Methacrylsäure und/oder Methacrylsäurederivaten oder Mischungen davon umfasst. Als retardierender Polymerfilm werden in erster Linie ein Polymerisat aus Methacrylsäure und Methacrylsäureestern wie Eudragit®L, Eudragit®S, ein Copolymerisat aus Acryl- und Methacrylsäureestern mit einem geringen Gehalt an Trimethylammoniummethacrylat wie Eudragit®RL, Eudragit®RS, ein Copolymerisat aus Acrylsäure, Methacrylsäure sowie deren Estern (Verhältnis der freien Carboxylgruppen zu den Estergruppen z.B. 1:1) wie Eudragit ®L30D oder ein Copolymerisat aus Acrylsäureethylund Methacrylsäuremethylester wie Eudragit®NE30D oder Mischungen aus diesen eingesetzt. Durch die Verwendung dieser Komponenten als Retardierkomponente wird eine gleichmässige, sichere und individuell einstellbare Retardierung erzielt.

Ganz besonders bevorzugt wird dabei ein quellbares, aber wasserunlösliches Ethylacrylat-Methylmethacrylat-Copolymer mit neutralen Estergruppen, wie Eudragit® NE30D, eingesetzt.

Beim Überziehen des kompaktierten Wirkstoffes mit der Retardkomponente werden ggf. zusätzliche pharmazeutisch übliche Hilfsstoffe, die die Freisetzung nicht beeinflussen, wie Trennmittel oder Pigmente, eingesetzt. Insbesondere Polymerfilme mit niedrigen Übergangstemperaturen wie z.B. Eudragit®NE30D zeigen eine hohe Klebeneigung. Dadurch können die Wirkstoffkompaktate beim Überziehen mit der Retardkomponente - beispielsweise in einem Wirbelschichtgerät - leicht aneinander kleben. Wiederholtes Verkleben und Trennen der Wirkstoffkompaktate während des Überziehens führt zu Fehlstellen im Überzug. Durch den Einsatz von Trennmitteln wie Talkum, Magnesiumstearat, Glycerolmonostearat, Calciumarachinat, Glycerolpalmitostearat, Stearinsäure und Triglyceriden wird dies verhindert. Eine reproduzierbare und kontrollierte Wirkstofffreisetzung wird so ermöglicht und das sog. "dose dumping" verhindert. Trennmittel bzw. Farbpigmente haben keinen Einfluss auf die Retardierungszeit.

Vorteilhaft kann die Zugabe von Hilfsstoffen wie Weichmachern, Tensiden, Antischaummittel, Emulgatoren und Porenbildnern bei der erfindungsgemäßen Arzneimittelzubereitung unterbleiben. Auch ohne Zugabe von externen Weichmachern wie z.B. Glyceroltriacetat, Tributylcitrat, Dibutylsebacat und Dibutylphthalat ist der Überzug elastisch genug, um mechanischen Kräften, die beispielsweise während einer nachfolgenden Tablettierung auftreten, zu widerstehen. Überraschenderweise kann ein Überzug ohne oder mit einer tolerierbaren Anzahl an Fehlstellen hergestellt und es können reproduzierbare Verzögerungszeiten erzielt werden, obwohl auf den Einsatz von Porenbildnern verzichtet wird. Porenbildner sind üblicherweise homogen im Überzug verteilt und werden im Freisetzungsmedium aus dem Filmüberzug herausgelöst, wodurch sich Poren bilden, durch die Flüssigkeit aus dem Freisetzungsmedium durch den in der Regel wenig permeablen Filmüberzug eindringen kann. Porenbildner wie z.B. Alkalisalze, mehrwertige Alkohole, Saccharose, Mannit, Sorbit, lösliche Polymere wie Carbopol, Polyethylenglycole usw. werden üblicherweise zur Regulation der Wirkstofffreisetzung und der Einstellung der Verzögerungszeiten eingesetzt.

Das Überziehen mit der Retardkomponente erfolgt wie allgemein bekannt, etwa durch Aufsprühen von Lösungen der Retardkomponente in organischen Lösungsmitteln oder Suspensionen der Retardkomponente in organischen Lösungsmitteln oder Wasser. Das Aufsprühen erfolgt z.B. im Luftsuspensionsverfahren (z.B. Glatt Wirbelschichtanlage WSG).

Gemäss einer besonders vorteilhaften Arzneimittelzubereitung beträgt in der Wirkstoffformulierung mit verzögerter Wirkstofffreisetzung das Verhältnis der Gewichtsteile von retardierendem Polymerfilm zu Flupirtin oder seinen physiologisch verträglichen Salzen zwischen 0,001 und 20, vorzugsweise zwischen 0,01 und 10, am meisten bevorzugt zwischen 0,05 und 0,1. Es wird somit eine ökonomische Herstellung von Arzneimittelzubereitungen in Form von sog. "multiple unit dosage forms" mit kontrollierter Freisetzung trotz nur geringer Mengen an retardierendem Polymerfilm erhalten.

Besonders von Vorteil ist eine erfindungsgemäße Arzneimittelzubereitung, wobei in vitro innerhalb von 240 Minuten zwischen 35 und 75 Prozent, vorzugsweise innerhalb von 15 Minuten zwischen 15 und 35 Prozent, noch bevorzugter innerhalb von 240 Minuten zwischen 55 und 75 Prozent und innerhalb von 600 Minuten 75 Prozent, des Flupirtins oder seiner physiologisch verträglichen Salze freigesetzt werden. Dadurch wird eine lang anhaltende und gleichmässige Abgabe von Flupirtin erzielt, die eine ein- oder zweimalige Einnahme der Arzneimittelzubereitung pro Tag ermöglicht.

Die in vitro - Freisetzungsgeschwindigkeit des Wirkstoffes aus der Arzneimittelzubereitung wird dabei unter Anwendung der Ph. Eur. Paddle Method bei 100 U/min in einem Puffer (gemäss Ph. Eur.) bei einem pH-Wert von 6,8 bei 37°C und unter UV-spektrophotometrischen Detektion, gemessen.

Vorzugsweise liegt ein Teil des Flupirtins oder seiner physiologisch verträglichen Salze als Wirkstoffformulierung mit sofortiger Wirkstofffreisetzung vor. Dabei ist die Wirkstoffformulierung mit sofortiger Wirkstofffreisetzung z.B. ohne Zusatz einer Retardkomponente formuliert und kann dabei kompaktiert oder nicht-kompaktiert oder auch in beiden Formen gleichzeitig vorliegen. Der Begriff "sofortige Wirkstofffreisetzung" bedeutet im Rahmen der vorliegenden Erfindung vorzugsweise eine in vitro Wirkstofffreisetzung von mindestens 80% innerhalb von 45 Minuten, besonders bevorzugt innerhalb von 30 Minuten. Diese in vitro Freisetzungsgeschwindigkeit des Wirkstoffes aus der Arzneimittelzubereitung wird dabei unter Anwendung der Ph. Eur. Paddle Method bei 75 U/min in 0,1 N Salzsäure bei 37°C und unter UV-spektrophotometrischen Detektion, gemessen.

Das als Wirkstoffformulierung mit sofortiger Wirkstofffreisetzung vorgesehene Flupirtin bzw. seine physiologisch verträglichen Salze dient als Initialphase und gewährleistet ein rasches Anfluten des Wirkstoffs im Plasma, was zu einer raschen Schmerzlinderung beim Patienten führt ("rapid onset"). Durch das als Wirkstoffformulierung mit verzögerter Wirkstofffreisetzung vorgesehene Flupirtin bzw. seine physiologisch verträglichen Salze wird gleichzeitig ein langanhaltender schmerzstillender Effekt über einen relativ langen Zeitraum (ca. 12 - 24 Stunden) erreicht. Somit kann mit einer einzigen Arzneimittelzubereitung eine für Schmerzmittel wünschenswerte schnell einsetzende und gleichzeitig lang anhaltende Wirkung erzielt werden. Die erfindungsgemässe Arzneimittelzubereitung vereinigt damit die Eigenschaften einer Formulierung mit sofortiger Wirkstofffreisetzung - schnelle Linderung des Schmerzes durch ausreichend hohe Wirkstoffkonzentration kurz nach Gabe des Arzneimittels - mit den Eigenschaften der Formulierung mit verzögerter Freisetzung - langandauemde analgetische Wirkung aufgrund eines ausreichend hohen Wirkstoffspiegels über längere Zeit. Der Schmerzpatient kann somit durch Einnahme der erfindungsgemäßen Arzneimittelzubereitung seine Schmerzen wirksam akut bekämpfen und zugleich ohne weitere Massnahmen und lediglich durch regelmässige Einnahme ein bis zweimal täglich effektiv über einen längeren Zeitraum therapieren.

Vorteilhafterweise beträgt das Verhältnis von Flupirtin als Wirkstoffformulierung mit verzögerter Wirkstofffreisetzung zu Flupirtin als Wirkstoffformulierung mit sofortiger Freisetzung zwischen 1 zu 2 und 9 zu 1, vorzugsweise 3 zu 1. Damit wird ein optimales Verhältnis zwischen beiden Flupirtin-Freisetzungsgeschwindigkeiten erzielt. Das genaue Verhältnis hängt von der Dosierungsstärke und von der erwünschten Abgabe ab.

Gemäss einer besonders vorteilhaften Ausführungsform liegt die Arzneimittelzubereitung in Form von Tabletten, Filmtabletten, Hartgelatinekapseln, Weichgelatinekapseln, Pellets, Granulaten, Dragees oder Mikrokapseln vor. Besonders bevorzugt ist eine Darreichung in Tablettenform. Oblonge Tablettenformen gestatten dabei die Teilbarkeit der Tablette, wodurch die Tabletten einfacher geschluckt werden können. Da die verzögerte Wirkstofffreisetzung aus den überzogenen Wirkstoffkompaktaten erfolgt, wird dadurch das Freisetzungsprofil nicht beeinflusst.

Die Gehalte an Flupirtin in den erfindungsgemässen Zubereitungen betragen dabei vorzugsweise 10 mg - 1000 mg, besonders bevorzugt 50 mg - 500 mg Flupirtin (bezogen auf die Base).

Vorzugsweise umfasst die Arzneimittelzubereitung weiters zumindest einen Außenphasenbestandteil. Dieser ist je nach Darreichungsform z.B. Einbettungsmittel, Sprengmittel, Fließregulierungsmittel und/oder Schmiermittel oder Mischungen davon. Dadurch können individuell einstellbare Eigenschaften der Formulierung erhalten werden.

Besonders bevorzugt umfasst der Außenphasenbestandteil Crosscarmellose-Na, mikrokristalline Cellulose, hochdisperses Siliciumdioxid, Magnesiumstearat, Pulvercellulose, Calciumhydrogenphosphat Dihydrat, Laktose, Mannitol und/oder Stärke oder Mischungen davon. Die Außenphasenbestandteile beeinflussen dabei vorzugsweise die Wirkstofffreisetzung nicht.

Einbettungsmittel wie mikrokristalline Cellulose sind aufgrund ihrer plastischen Verformbarkeit und ihrer hohen Porosität in der Lage, die Wirkstoffformulierung mit verzögerter Freisetzung vor zu starken mechanischen Belastungen zu schützen und die bei der Herstellung der jeweiligen Darreichungsformen der Arzneimittelzusammensetzung wirkenden Kräfte abzupuffern. Insbesondere die Verpressung zu Tabletten stellt für die Wirkstoffformulierung mit verzögerter Wirkstofffreisetzung eine erhebliche mechanische Belastung dar: Das angestrebte Freisetzungsverhalten der Arzneimittelzubereitung kann verlorengehen, falls beispielsweise während der Tablettierung der Wirkstoffformulierung mit verzögerter Wirkstofffreisetzung der aus dem retardierenden Polymerfilm und ggf. Hilfsstoffen bestehende Überzug beschädigt wird. Um dies zu verhindern, werden z.B. insbesondere bei der Tablettierung vorzugsweise zusätzlich Einbettungmittel zugesetzt.

Liegt in der erfindungsgemäßen Arzneimittelzubereitung neben der Wirkstoffformulierung mit verzögerter Wirkstofffreisetzung zusätzlich ein Teil des enhaltenen Flupirtins bzw. seiner physiologisch verträglichen Salze als Wirkstoffformulierung mit sofortiger Wirkstofffreisetzung vor, so sind in der Darreichungsform die Wirkstoffformulierung mit verzögerter Wirkstofffreisetzung und die Wirkstoffformulierung mit sofortiger Wirkstofffreisetzung gegebenenfalls unter Zusatz von Außenphasebestandteilen vorzugsweise homogen miteinander vermischt. Dadurch kann die Arzneimittelzubereitung in der Dosierung modifiziert werden, ohne die Wirkstofffreisetzung zu beeinflussen.

Gemäss einem weiteren Aspekt der Erfindung wird ein Wirkstoffkompaktat umfassend Flupirtin zur Verfügung gestellt, das eine Teilchengröße von 160 bis 800 µm, vorzugsweise 250 bis 500 µm, aufweist und bevorzugt sphärisch oder annähernd sphärisch ist. Dabei ist der Begriff "Wirkstoffkompaktat" nicht nur als Singular sondern auch als Plural zu verstehen, so dass mehrere Wirkstoffkompaktate ebenfalls hiervon mitumfasst sind. Diese Wirkstoffkompaktate dienen insbesondere zur Herstellung der erfindungsgemässen Arzneimittelzubereitung. Hierbei gelten die oben genannten Definitionen und bevorzugten Ausführungsformen.

Dabei ist es besonders vorteilhaft, wenn das Wirkstoffkompaktat mit einer Retardkomponente überzogen ist, die vorzugsweise einen retardierenden Polymerfilm und ggf. übliche pharmazeutische Hilfsstoffe umfasst.

Vorzugsweise weist das Wirkstoffkompaktat ein Schüttvolumen von unter 5 ml/g, vorzugsweise unter 3 ml/g, noch bevorzugter unter 2,5 ml/g, am meisten bevorzugt zwischen 0,8 und 2,5 ml/g, auf.

Wie oben beschrieben, wird durch die Kompaktierung des Flupirtins der Wirkstoff sphärischer oder annähernd sphärischer Morphologie mit gleichmässiger Größenverteilung erhalten, so dass eine reproduzierbare und einheitliche Beschichtung des Flupirtins mit Retardkomponenten und eine genaue Dosierung sowie eine gleichmäßige Freisetzung des Wirkstoffes aus dem Kompaktat erzielt wird.

Ein weiterer Gegenstand der vorliegenden Erfindung ist ein Verfahren zur Herstellung einer Arzneimittelzubereitung wie oben beschrieben, wobei
a. Flupirtin oder seine physiologisch verträglichen Salze, gegebenenfalls mit pharmazeutisch üblichen Hilfsstoffen, zu Wirkstoffkompaktaten kompaktiert werden,
b. Wirkstoffkompaktate mit einer Teilchengröße von 160 bis 800 µm separiert werden und
c. die separierten Wirkstoffkompaktate mit einer Retardkomponente überzogen werden.

Hierbei gelten wiederum die oben angeführten Definitionen und bevorzugten Ausführungsformen. Wie oben beschrieben, wird durch die Kompaktierung des Flupirtins der Wirkstoff sphärischer oder annähernd sphärischer Morphologie mit gleichmässiger Größenverteilung erhalten, so dass eine reproduzierbare und einheitliche Beschichtung des Flupirtins mit Retardkomponenten und eine genaue Dosierung sowie eine gleichmäßige Freisetzung des Wirkstoffes aus dem Kompaktat erzielt wird.

Gemäss einer vorteilhaften Ausführungsform werden die Wirkstoffkompaktate bei Verwendung von Retardkomponenten mit einer hohen Klebeneignung zusätzlich mit einem Trennmittel überzogen, so dass im Überzug Fehlstellen vermieden werden. Dadurch wird eine reproduzierbare und kontrollierte Wirkstofffreisetzung ermöglicht. Bei diesem Verfahren gelten die Definitionen und bevorzugten Ausführungsformen wie oben für die Arzneimittelzubereitung beschrieben.

Besonders bevorzugt werden die Wirkstoffkompaktate, gegebenenfalls unter Zusatz von Außenphasenbestandteilen, in pharmazeutisch üblicher Weise zu einer Arzneimittelzubereitung, vorzugsweise zu Tabletten, Filmtabletten, Hartgelatinekapseln, Weichgelatinekapseln, Pellets, Granulaten, Dragees oder Mikrokapseln, weiterverarbeitet.

Die erfindungsgemässe Arzneimittelzubereitung wird nun anhand der nachfolgenden Beispiele und Figuren 1 bis 3 näher erläutert.

Dabei zeigen
Fig. 1A - 1D die Morphologie des Wirkstoffs Flupirtin vor der Kompaktierung
Fig. 2A - 2C die Morphologie des Wirkstoffs Flupirtin nach Kompaktierung;
Figur 3 ein Diagramm der in vivo Freisetzung: Konzentration von Flupirtin in µg/ml gegen die Zeit.

### Beispiel 1

### Morphologie des Flupirtins

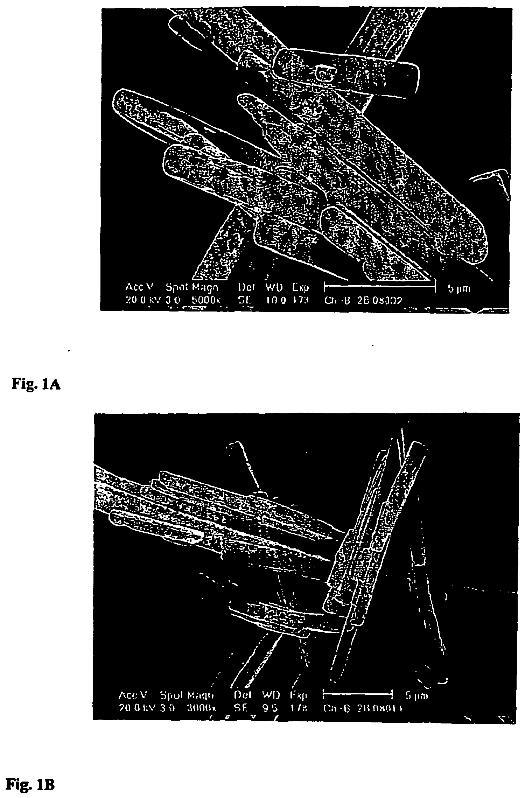

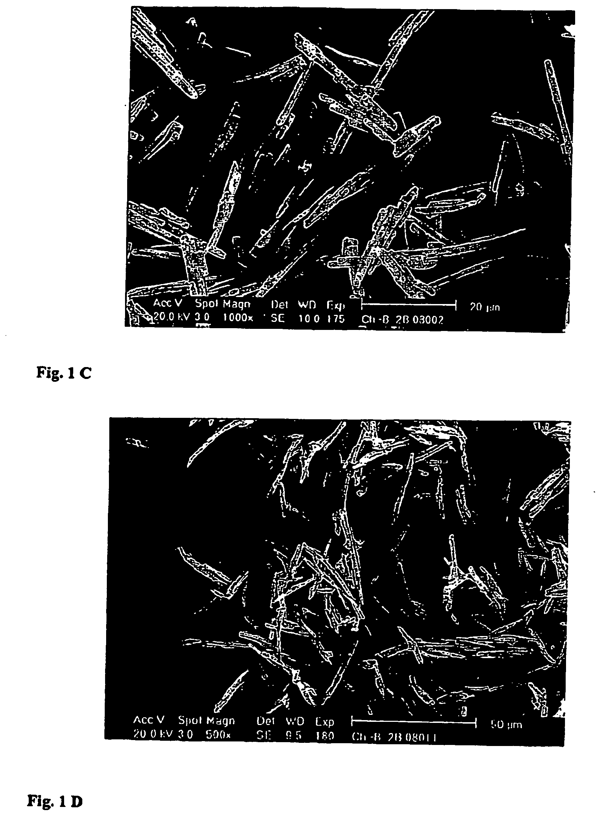

Flupirtinmaleat Kristalle wurden abgebildet und sind in Fig 1A - 1D (vier Abbildungen) in verschiedenen Grössen gezeigt. Die nadelförmige Morphologie ist deutlich zu erkennen.

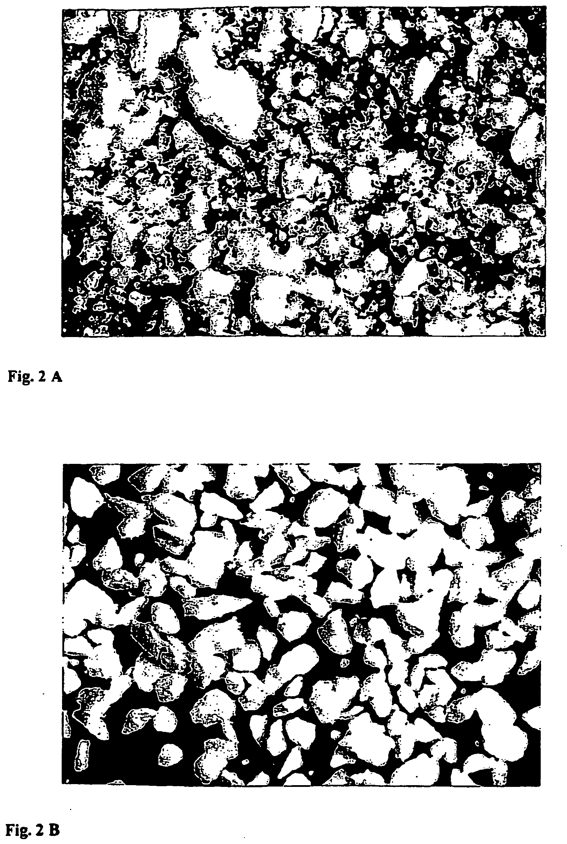

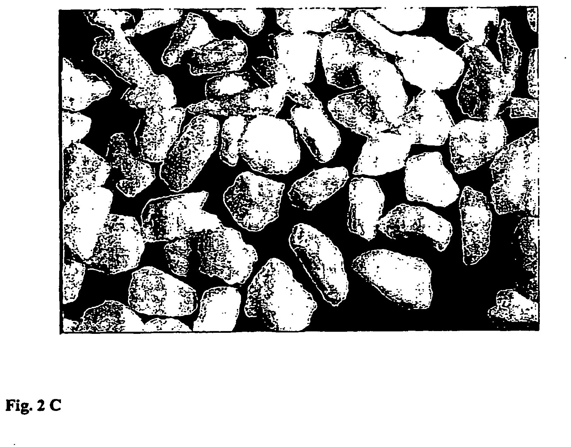

Diese Kristalle wurden anschliessend kompaktiert (Kompaktor Alexanderwerk) und nach Teilchengrösse separiert. Fig. 2A zeigt die Kompaktate ohne Separierung, Fig. 2B zeigt die Fraktion der Kompaktate mit 200 - 400µm und Fig. 2C zeigt die Fraktion der Kompaktate über 400µm Durchmesser.

Es wird verdeutlicht, dass die Morphologie des Flupirtins erst nach Kompaktierung und Separierung oder Fraktionierung eine gleichmässige und reproduzierbare Beschichtung mit einer Retardkomponente erlaubt.

### Beispiel 2

### Flupirtinmaleat umfassende Tablette mit verzögerter Wirkstofffreisetzung

Der Wirkstoff Flupirtinmaleat wird in einem Trockengranulator (Walzenpresse) verdichtet und gebrochen und anschließend wird mittels einer Klassiersiebmaschine in einer Korngröße von 250 bis 500 µm fraktioniert.

13 kg kompaktiertes Flupirtinmaleat wird in einem Wirbelschichtgranulator mit 3,25 kg einer wässrigen Suspension aus Poly(ethylacrylat-methylmethacrylat)-Dispersion 30 % (Eudragit® NE 30 D), 0,975kg Talkum und 0,098 kg des Färbemittels Eisenoxid gelb befilmt. Das Granulat wird anschließend getrocknet, wobei die Produkttemperatur maximal 35°C beträgt. Dieses Granulat wird mit 0,063 kg Hochdispersem Siliciumdioxid im Wirbelschichtgranulator gemischt und anschließend über eine Pendelsiebmaschine (Siebmaschenweite 1 mm) gesiebt.

### Beispiel 3

### Flupirtinmaleat umfassende Tablette mit einem Anteil mit sofortiger Wirkstofffreisetzung und einem Anteil mit verzögerter Wirkstofffreisetzung

Für die Herstellung von 13,896 kg Granulat mit sofortiger Wirkstofffreisetzung wird 7,2 kg Flupirtinmaleat mit 2,736 kg Calciumhydrogenphosphat-Dihydrat, 0,36 kg Crosscarmellose-Na, 2,376 kg Mikrokristalliner Cellulose und 0,288 kg Eisenoxid gelb in einem Diffusionsmischer vorgemischt und danach wird die Mischung mit 0,576 kg Methylhydroxypropylcellulose in Gereinigtem Wasser in einem High-Shear Granulator granuliert und anschließend in einem Wirbelschichttrockner getrocknet, wobei die Produkttemperatur maximal 35°C beträgt. Das getrocknete Granulat mit sofortiger Wirkstofffreisetzung wird mittels Pendelsiebmaschine (Siebmaschenweite: 1 mm) gesiebt.

15 kg von laut Ausführungsbeispiel 2 hergestelltem Granulat mit verzögerter Wirkstofffreisetzung und 8,302 kg Granulat mit sofortiger Wirkstofffreisetzung werden mit 1,288 kg Crosscarmellose-Na, 0,935 kg Mikrokristalline Cellulose, 0,026 kg Hochdispersem Siliciumdioxid und 0,258 kg Magnesiumstearat in einem Diffusionsmischer homogenisiert.

Auf einer Rundläufertablettenpresse wird die Tablettiermischung zu gewölbten Oblong Tabletten von 16 mm Länge und 7 mm Breite, mit Bruchkerbe auf einer Seite verarbeitet.

Die hergestellten Tabletten wurden neben einer *in-vitro* auch einer *in-vivo* Prüfung unterzogen. In einer 3 fach crossover Untersuchung an 24 gesunden Probanden wurde das kinetische Profil und die Bioverfügbarkeit dieser Tablette ("Test") mit der herkömmlichen Kapselformulierung mit nicht kontrollierter Wirkstofffreisetzung ("Referenz") nach einer 1x Gabe verglichen. Dabei wurden 4 Kapseln der Referenzformulierung (= 400 mg Flupirtinmaleat) bzw. eine Tablette der Testformulierung (= 400 mg Flupirtinmaleat im Verhältnis 100 mg mit sofortiger und 300 mg mit verzögerter Wirkstofffreisetzung) unter Nüchternbedingungen appliziert und der Plasmakonzentrationsverlauf über 48 h (Tfa) bzw. 36 h (Rfa) bestimmt. Weiterhin wurde der Einfluß der Nahrung auf die Kinetik der neuen Arzneimittelzubereitung (Tfe) über 48 h untersucht.

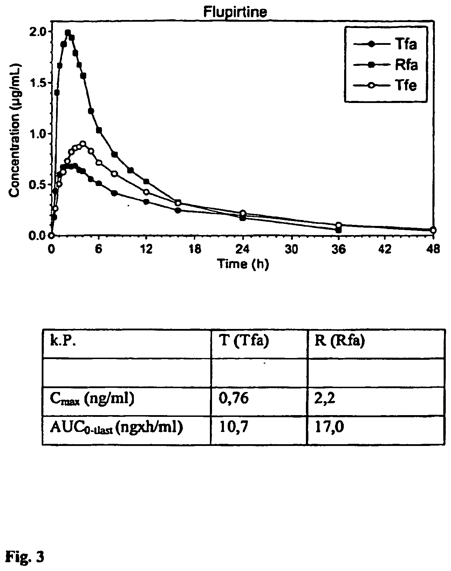

Die erhaltenen Verlaufskurven des Blutspiegels von Flupirtin sind in Fig. 3 dargestellt. Das Konzentrationsmaximum (Cₘₐₓ) der "Test"-Zubereitung (Tfa) beträgt erwartungsgemäß 25% des Konzentrationsmaximums der Referenz-Kapselzubereitung (Rfa) als Ausdruck der raschen Absorption des Anteils mit sofortiger Wirkstoffreisetzung der Tablette (s. Tabelle in Fig. 3, wobei k.P. "kinetischer Parameter", T "Test" und R "Referenz" bedeuten).

Die Bioverfügbarkeit des Wirkstoffes (bestimmt über die Fläche unter der Blutspiegelkurve innerhalb des Beobachtungszeitraumes [AUC_{0-tlast.}]) beträgt 63 % (Bereich 59-67%). Dennoch ist ein therapeutisch relevanter Blutspiegel über 24 Stunden nachweisbar. Der Verlauf der Flupirtin-Konzentration unter Nahrungseinfluß (Tfe) ist geringfügig höher als unter Nüchternbedingungen (Tfa). Dies ist aber nicht durch die geänderte Galenik bedingt sondern als Substanzeigenschaft bekannt.

Die relative Häufigkeit der angegebenen Nebenwirkungen ist für die Test-Verbindung mit 8% (= 10 Nebenwirkungen) im Vergleich zu 75% bei der Kapselformulierung (= 89 Nebenwirkungen) eindeutig geringer und bestätigt des Konzept einer Reduktion der unerwünschten Effekte, wenn die Art des Einstromes des Wirkstoffes in die systemische Zirkulation geändert wird.

## Patentansprüche

1. Feste Arzneimittelzubereitung, umfassend Flupirtin oder seine physiologisch verträglichen Salze als Wirkstoff, wobei mindestens ein Teil des Flupirtins oder seiner physiologisch verträglichen Salze als Wirkstoffformulierung mit verzögerter Wirkstofffreisetzung vorliegt, **dadurch gekennzeichnet, dass**
a. die Wirkstoffformulierung mit verzögerter Wirkstofffreisetzung Wirkstoffkompaktate umfasst, die mit einer Retardkomponente, vorzugsweise gleichmässig, überzogen sind und gegebenenfalls pharmazeutisch übliche Hilfsstoffe umfassen, so dass eine gleichmässige Freisetzung des Flupirtins aus den Kompaktaten gewährleistet ist, und
b. die Wirkstoffkompaktate eine Teilchengröße von 160 bis 800 µm, vorzugsweise 250 bis 500 µm, aufweisen und bevorzugt sphärisch oder annähernd sphärisch sind.

2. Arzneimittelzubereitung nach Anspruch 1, **dadurch gekennzeichnet, dass** die Wirkstoffkompaktate ein Schüttvolumen von unter 5 ml/g, vorzugsweise unter 3 ml/g, noch bevorzugter unter 2.5 ml/g, am meisten bevorzugt zwischen 0.8 und 2.5 ml/g, aufweisen.

3. Arzneimittelzubereitung nach Anspruch 1 oder 2, **dadurch gekennzeichnet, dass** die Retardkomponente eine diffusionskontrollierte Freisetzung des Wirkstoffes gewährleistet.

4. Arzneimittelzubereitung nach einem der Ansprüche 1 bis 3, **dadurch gekennzeichnet, dass** die Retardkomponente einen retardierenden Polymerfilm und ggf. übliche pharmazeutische Hilfsstoffe, vorzugsweise Trennmittel und Pigmente, umfasst, wobei der retardierende Polymerfilm vorzugsweise zumindest ein Polymer oder Copolymer aus Acrylsäure, Acrylsäurederivaten, Methacrylsäure und/oder Methacrylsäurederivaten oder Mischungen davon umfasst.

5. Arzneimittelzubereitung nach Anspruch 4, **dadurch gekennzeichnet, dass** in der Wirkstoffformulierung mit verzögerter Wirkstofffreisetzung das Verhältnis der Gewichtsteile von retardierendem Polymerfilm zu Flupirtin oder seinen physiologisch verträglichen Salzen zwischen 0,001 und 20, vorzugsweise zwischen 0,01 und 10, am meisten bevorzugt zwischen 0,05 und 0,1 beträgt.

6. Arzneimittelzubereitung nach einem der Ansprüche 1 bis 5, **dadurch gekennzeichnet, dass** in vitro innerhalb von 240 Minuten zwischen 35 und 75 Prozent, vorzugsweise innerhalb von 15 Minuten zwischen 15 und 35 Prozent, noch bevorzugter innerhalb von 240 Minuten zwischen 55 und 75 Prozent und innerhalb von 600 Minuten 75 Prozent, des Flupirtins oder seiner physiologisch verträglichen Salze freigesetzt werden.

7. Arzneimittelzubereitung nach einem der Ansprüche 1 bis 6, **dadurch gekennzeichnet, dass** ein Teil des Flupirtins oder seiner physiologisch verträglichen Salze als Wirkstoffformulierung mit sofortiger Wirkstofffreisetzung vorliegt.

8. Arzneimittelzubereitung nach Anspruch 7, **dadurch gekennzeichnet, dass** das Verhältnis von Flupirtin als Wirkstoffformulierung mit verzögerter Wirkstofffreisetzung zu Flupirtin als Wirkstoffformulierung mit sofortiger Freisetzung zwischen 1 zu 2 und 9 zu 1, vorzugsweise 3 zu 1, beträgt.

9. Arzneimittelzubereitung nach einem der Ansprüche 1 bis 8 **dadurch gekennzeichnet, dass** sie in Form von Tabletten, Filmtabletten, Hartgelatinekapseln, Weichgelatinekapseln, Pellets, Granulaten, Dragees oder Mikrokapseln vorliegt.

10. Arzneimittelzubereitung nach einem der Ansprüche 1 bis 9, **dadurch gekennzeichnet, dass** sie weiters zumindest einen Außenphasenbestandteil umfasst.

11. Arzneimittelzubereitung nach Anspruch 10, **dadurch gekennzeichnet, dass** der Außenphasenbestandteil Crosscarmellose-Na, mikrokristalline Cellulose, hochdisperses Siliciumdioxid, Magnesiumstearat, Pulvercellulose, Calciumhydrogenphosphat Dihydrat, Laktose, Mannitol und/oder Stärke oder Mischungen davon umfasst.

12. Arzneimittelzubereitung nach einem der Ansprüche 1 bis 11, **dadurch gekennzeichnet, dass** sie zerteilbar ist.

13. Verfahren zur Herstellung einer Arzneimittelzubereitung nach einem der Ansprüche 1 bis 12, **dadurch gekennzeichnet, dass**
a. Flupirtin oder seine physiologisch verträglichen Salze, gegebenenfalls mit pharmazeutisch üblichen Hilfsstoffen, zu Wirkstoffkompaktaten kompaktiert werden,
b. Wirkstoffkompaktate mit einer Teilchengröße von 160 bis 800 µm separiert werden und
c. die separierten Wirkstoffkompaktate mit einer Retardkomponente überzogen werden.

14. Verfahren nach Anspruch 13, **dadurch gekennzeichnet, dass** bei Verwendung einer Retardkomponente mit hoher Klebeneigung zusätzlich ein Überzug mit Trennmittel erfolgt, so dass im Überzug Fehlstellen vermieden werden.

15. Verfahren nach Anspruch 13 oder 14, **dadurch gekennzeichnet, dass** die Wirkstoffkompaktate, gegebenenfalls unter Zusatz von Außenphasenbestandteilen, in pharmazeutisch üblicher Weise zu einer Arzneimittelzubereitung, vorzugsweise zu Tabletten, Filmtabletten, Hartgelatinekapseln, Weichgelatinekapseln, Pellets, Granulaten, Dragees oder Mikrokapseln oder Suppositorien weiterverarbeitet werden.

## Claims

1. Solid pharmaceutical preparation, comprising Flupirtin or its physiologically compatible salts as active ingredient, whereby at least one portion of the Flupirtin or its physiologically compatible salts is present as an active ingredient formulation with a delayed active ingredient release, **characterized in that**
a. the active ingredient formulation with delayed active ingredient release includes active ingredient compactates that are, preferably evenly, covered with a retard component and include common pharmaceutical excipients if necessary, so that a steady release of the Flupirtin out of the compactates is guaranteed, and
b. the active ingredient compactates have a particle size of 160 to 800 µm, preferably 250 to 500 µm, and are preferably spherical or approximately spherical.

2. Pharmaceutical preparation according to Claim 1, **characterized in that** the active ingredient compactates have a bulk volume of less than 5 ml/g, preferably less than 3 ml/g, even more preferably less than 2.5 ml/g, most preferably between 0.8 and 2.5 ml/g.

3. Pharmaceutical preparation according to one of Claims 1 or 2, **characterized in that** the retard component guarantees a diffusion controlled release of the active ingredient.

4. Pharmaceutical preparation according to one of the Claims 1 to 3, **characterized in that** the retard component includes a retarding polymer film and common pharmaceutical excipients, preferably release agents and pigments, if necessary, whereby the retarding polymer film preferably includes at least one polymer or copolymer made of acrylic acid, acrylic acid derivatives, methacrylic acid and/or methacrylic acid derivatives or a mixture thereof.

5. Pharmaceutical preparation according to Claim 4, **characterized in that**, in the active ingredient formulation with delayed active ingredient release, the ratio of the part by weight of retarding polymer film to Flupirtin or its physiologically compatible salts is between 0.001 and 20, preferably between 0.01 and 10 and most preferably between 0.05 and 0.1.

6. Pharmaceutical preparation according to one of Claims 1 to 5, **characterized in that** in vitro within 240 minutes between 35 and 75 percent, preferably within 15 minutes between 15 and 35 percent, even more preferably within 240 minutes between 55 and 75 percent and within 600 minutes 75 percent, of the Flupritin or its physiological compatible salts are released.

7. Pharmaceutical preparation according to one of Claims 1 to 6, **characterized in that** a portion of the Flupritin or its physiological compatible salts is present as an active ingredient formulation with immediate active ingredient release.

8. Pharmaceutical preparation according to Claim 7, **characterized in that** the ratio of Flupirtin as an active ingredient formulation with delayed active ingredient release to Flupirtin as an active ingredient formulation with immediate active ingredient release is between 1 to 2 and 9 to 1, preferably 3 to 1.

9. Pharmaceutical preparation according to one of the Claims 1 to 8, **characterized in that** it is present in the form of tablets, film-coated tablets, hard gelatin capsules, soft gelatin capsules, pellets, granules, coated tables or microcapsules.

10. Pharmaceutical preparation according to one of Claims 1 to 9, **characterized in that** it furthermore includes at least one external phase component.

11. Pharmaceutical preparation according to Claim 10, **characterized in that** the external phase component includes croscarmellose sodium, microcrystalline cellulose, highly dispersed silicon dioxide, magnesium stearate, powder cellulose, calcium hydrogen phosphate dihydrate, lactose, mannitol and/or starch or mixtures thereof.

12. Pharmaceutical preparation according to one of Claims 1 to 11, **characterized in that** it is split able.

13. Method for producing a pharmaceutical preparation according to one of Claims 1 to 12, **characterized in that**
a. Flupirtin or its physiologically compatible salts are compacted to active ingredient compactates, with the pharmaceutically common additives if necessary,
b. active ingredient compactates with a particle size of 160 to 180 µm are separated, and
c. the separated active ingredient compactates are covered with a retard component.

14. Method according to Claim 13, **characterized in that** when using a retard component with strong adhesive tendencies, a coating with a release agent follows in addition, so that imperfection in the coating are avoided.

15. Method according to Claim 13 or 14, **characterized in that** the active ingredient compactates are further processed, with the addition of external phase components if necessary, into a pharmaceutical preparation, preferably into tablets, film-coated tables, hard gelatin capsules, soft gelatin capsules pellets, granules or microcapsules or suppositories, in a pharmaceutically common manner.

## Revendications

1. Préparation médicamenteuse solide comprenant en tant que principe actif de la flupirtine ou ses sels physiologiquement compatibles, au moins une partie de la flupirtine ou de ses sels physiologiquement compatibles étant présente en tant que formulation de principe actif à libération retardée du principe actif, **caractérisée en ce que**
a. la formulation de principe actif présentant une libération retardée du principe actif comprend des agglomérats de principe actif présentant des granulométries distinctes, qui sont enrobés d'un composant retard, de préférence uniformément, et éventuellement comprennent des adjuvants pharmaceutiquement usuels, de façon à garantir une libération uniforme de la flupirtine à partir des agglomérats, et
b. les agglomérats de principe actif présentent une granulométrie de 160 à 800 µm, de préférence de 250 à 500 µm, et ont de préférence une forme sphérique ou approximativement sphérique.

2. Préparation médicamenteuse selon la revendication 1, **caractérisée en ce que** les agglomérats de principe actif ont un volume massique avant tassement inférieur à 5 ml/g, de préférence inférieur à 3 ml/g, d'une manière encore plus préférée inférieure à 2,5 ml/g, de la manière la plus préférée compris entre 0,8 et 2,5 ml/g.

3. Préparation médicamenteuse selon la revendication 1 ou 2, **caractérisée en ce que** le composant retard assure une libération du principe actif contrôlée par diffusion.

4. Préparation médicamenteuse selon l'une des revendications 1 à 3, **caractérisée en ce que** le composant retard comprend un film polymère à effet retard et éventuellement des adjuvants pharmaceutiques usuels, de préférence des lubrifiants et des pigments, le film polymère à effet retard comprenant de préférence au moins un polymère ou copolymère de l'acide acrylique, de dérivés de l'acide acrylique, de l'acide méthacrylique et/ou de dérivés de l'acide méthacrylique, ou de mélanges de ceux-ci.

5. Préparation médicamenteuse selon la revendication 4, **caractérisée en ce que**, dans la formulation de principe actif présentant une libération retardée du principe actif, le rapport entre le nombre de parties en poids du film polymère à effet retard et celui de la flupirtine ou de ses sels physiologiquement compatibles, est compris entre 0,001 et 20, de préférence entre 0,01 et 10, de la manière la plus préférée entre 0,05 et 0,1.

6. Préparation médicamenteuse selon l'une des revendications 1 à 5, **caractérisée en ce que**, in vitro, en 240 minutes de 35 à 75 %, de préférence en 15 minutes de 15 à 35 %, d'une manière encore plus préférée en 240 minutes de 55 à 75 %, et en 600 minutes 75 % de la flupirtine ou de ses sels physiologiquement compatibles sont libérés.

7. Préparation médicamenteuse selon l'une des revendications 1 à 6, **caractérisée en ce qu'**une partie de la flupirtine ou de ses sels physiologiquement compatibles est présente sous forme d'une formulation de principe actif présentant une libération immédiate du principe actif.

8. Préparation médicamenteuse selon la revendication 7, **caractérisée en ce que** le rapport de la flupirtine en tant que formulation de principe actif présentant une libération retardée du principe actif et la flupirtine en tant que formulation de principe actif présentant une libération immédiate, est compris entre 1:2 et 9:1, et est de préférence de 3:1.

9. Préparation médicamenteuse selon l'une des revendications 1 à 8, **caractérisée en ce qu'**elle se présente sous forme de comprimés, de comprimés enrobés, de gélules en gélatine dure, de capsules en gélatine molle, de pastilles, de granulés, de dragées ou de microcapsules.

10. Préparation médicamenteuse selon l'une des revendications 1 à 9, **caractérisée en ce qu'**elle comprend en outre au moins un constituant en phase extérieure.

11. Préparation médicamenteuse selon la revendication 10, **caractérisée en ce que** le constituant en phase extérieure comprend la croscarmellose-Na, la cellulose microcristalline, le dioxyde de silicium hautement dispersé, le stéarate de magnésium, la cellulose en poudre, l'hydrogénophosphate de calcium dihydraté, le lactose, le mannitol et/ou l'amidon, ou les mélanges de ceux-ci.

12. Préparation médicamenteuse selon l'une des revendications 1 à 11, **caractérisée en ce qu'**elle est sécable.

13. Procédé de fabrication d'une préparation médicamenteuse selon l'une des revendications 1 à 12, **caractérisé en ce que**
a. on compacte, pour obtenir des agglomérats de principe actif, de la flupirtine ou ses sels physiologiquement compatibles, éventuellement avec des adjuvants pharmaceutiquement usuels,
b. on sépare les agglomérats de principe actif ayant une granulométrie de 160 à 800 µm, et
c. on enrobe d'un composant retard les agglomérats de principe actif séparés.

14. Procédé selon la revendication 13, **caractérisé en ce que**, lors de l'utilisation d'un composant retard présentant une forte tendance à l'adhérence, on procède en outre à un enrobage d'un lubrifiant, de façon à éviter les solutions de continuité dans l'enrobage.

15. Procédé selon la revendication 13 ou 14, **caractérisé en ce que** les agglomérats de principe actif sont, éventuellement avec l'addition de constituants en phase extérieure, soumis à une post-transformation d'une manière usuelle en galénique, pour donner une préparation médicamenteuse, de préférence des comprimés, des comprimés enrobés, des gélules en gélatine dure, des capsules en gélatine molle, des pastilles, des granulés, des dragées ou des microcapsules ou des suppositoires.
